# EUROPEAN PATENT APPLICATION

(11) **EP 2 000 145 A2**
(43) Date of publication of application: **10.12.2008**
(21) Application number: 07739428.6
(22) Date of filing: 23.03.2007
(51) Int. Cl.: A61K 36/49, A23L 1/30, A61P 3/04, A61P 3/06, A61P 43/00

(54) **AMELIORATING AGENT FOR METABOLIC SYNDROME**

(30) Priority: 24.03.2006 JP 2006083003
(71) Applicant: Rohto Pharmaceutical Co., Ltd., Ikuno-ku Osaka-shi Osaka 544-8666 (JP)
(72) Inventor: KUWAHARA, Akiko, Osaka-shi, Osaka 544-8666 (JP); NAKANISHI, Ayako, Osaka-shi, Osaka 544-8666 (JP); SUDA, Kazuma, Osaka-shi, Osaka 544-8666 (JP)
(74) Representative: Beckmann, Claus
(86) International application number: PCT/JP2007/055987
(87) International publication number: WO 2007/111242

(57) **Abstract**

The object is to provide a food-derived effective ingredient which can promote the reduction in accumulated fats or the like. Disclosed is a body fat reduction-promoting agent, a leptin production-promoting agent, an anorexigenic agent, a PAI-1 production-suppressing agent or an anti-thrombotic agent, each comprising an extract from a chestnut astringent skin as an active ingredient. The extract from a chestnut astringent skin, which is a natural component, can be used as an extremely safe body fat reduction-promoting agent, leptin production-promoting agent, anorexigenic agent, PAI-1 production-suppressing agent or anti-thrombotic agent.
Particularly, the body fat reduction-promoting agent can reduce an accumulated visceral fat and therefore can directly prevent obesity.

## Description

### Technical Field

The present invention relates to a composition for reducing body fat and thus solving obesity, a composition for promoting leptinproduction and thus suppressing food intake, a composition for suppressing the production of plasminogen activator inhibitor-1 (hereinafter, referred to as PAI-1) and thus suppressing thrombus formation, and the like.

### Background Art

In the developed countries in Europe and the United States as well as Japan, obesity has been remarkably increasing as a result of hypernutrition due to excessive intake of animal proteins, animal fats, instant foods, fast foods and the like, and also lack of exercise.

Obesity is a state in which fats are stored or accumulated in excess in the body, including the subcutaneous adipose tissues, and in general, the case where the BMI value is 25 or higher, is classified as obesity.

Obesity is one of the risk factors for diabetes mellitus, hypertension, arteriosclerosis and hyperlipidemia, and is also said to be a cause of hyperuricemia, cholelithiasis, gout and the like.

In order to solve obesity, diet therapy based on calorie restriction, kinesitherapy based on energy consumption, therapy based on intake of food and beverage products or pharmaceutical products which are said to have anti-obesity effects, or a combination thereof may be employed. However, although the diet therapy and kinesitherapy in particular are expected to have certain effects, these methods need to be continued for a long time, and thus those who cannot endure this come to face a problem of weight rebound which can often be seen with transient weight loss.

Furthermore, drug therapy using an anorexiant such as an adrenalin agonist drug or serotonin agonist drug, also has problems from the aspects that the subject of application may be limited, such that drug therapy is allowed only to morbidly obese patients having difficulties with diet therapy, and that dependency is accompanied.

As shown above, intake of food and beverage products which have an anti-obesity effect and can be taken in more safely over long periods, is expected to be an effective method for solving obesity.

In fact, a truly wide variety of food and beverage products are reported to have an anti-obesity effect. As one of the mainstream classes, food and beverage products having a lipase inhibitory action (for example, Patent Documents 1 to 5, and the like).

A lipase is a lipid-digesting enzyme secreted from the pancreas in humans, which sequentiallyhydrolyzes the esterbonds of triacylglycerol, to sequentially produce diacylglycerol, monoacylglycerol, and then glycerol and fatty acids. Since it is suspected that excessive intake and absorption of the glycerol and fatty acids generated under the action of this lipase are cause of obesity, food and beverage products exhibiting a lipase activity inhibitory action are expected to be able to suppress the absorption of glycerol and fatty acids, and to consequently prevent obesity.

As an attempt to suppress the absorption of sugars, which is one of calorie sources, food and beverage products having a glucosidase inhibitory action are also being examined (for example, Patent Document 6). In the human intestines, polysaccharides or oligosaccharides are not absorbed, and absorption thereof begins for the first time only when the polysaccharides and oligosaccharides are degraded to monosaccharides by degradative enzymes such as glucosidases. Thus, it is expected that if these glucosidases exhibiting an oligosaccharide-degrading activity are inhibited, absorption of sugars is suppressed and accumulation of depot fat can be prevented, and therefore, it has been proposed to use a glucosidase inhibitor as an anti-obesity agent.

These attempts are attempts merely intended to prevent or suppress the accumulation of fat, and do not provide direct reduction of fat that has already been accumulated.

Moreover, in recent years, physiologically active substances called adipokines, which are produced and secreted by adipocytes, have been attracting public attention. As for the adipokines, leptin, PAI-1, TNF-α, HB-EGF, resistin, adiponectin and the like are known so far, and it has been suggested that these substances are deeply associated with various physiological functions, including sugar metabolism and lipid metabolism.

Among them, leptin is also referred to as a good cytokine, and it has been revealed that leptin exerts its action on leptin receptors to exhibit a strong anorexigenic effect, that leptin can exhibit an energy consumption enhancing effect, and the like (Non-Patent Document 1). Furthermore, it is reported that leptin can extensively participate in the regulation of physiological functions, such as by exhibiting a sugarmetabolism ameliorating action through activation of the sympathetic nervous system. Therefore, increasing the amount of production of leptin in the living body can be greatly helpful in solving obesity, and eventually in ameliorating metabolic syndrome.

On the other hand, PAI-1 is also referred to as a bad cytokine, and is a very important fibrinolysis (thrombolytic reaction) controlling factor in the onset of thrombosis (Non-Patent Document 2). At the site of thrombus formation, PAI-1 suppresses plasmin production by inhibiting the function of the plasminogen activator, stabilizes fibrin thrombus, and inhibits the lysis of extracellular matrix to thus promote thrombus deposition on the blood vessel walls.

It is conceived that in obese people, an increase in the PAI-1 concentration in blood caused by PAI-1 that is secreted by adipose tissues, as a result of an increment in the amount of adipose tissues, serves as an important factor of the onset of thrombosis. In recent years, severe thrombosis associated with obesity, hyperlipidemia, diabetes mellitus, hypertension and the like, is presenting problems. Thus, decreasing the amount of production of PAI-1 which participates in thrombus formation in the living body can be greatly helpful in suppressing the thrombus formation developing in association with obesity, and consequently metabolic syndrome. Furthermore, it has been suggested that PAI-1 is related also to obesity per se or insulin resistance, in addition to thrombus formation, and thus the anti-obesity effects or an improvement in insulin resistance through suppression of the amount of production of PAI-1, is also expected.

As one of the lifestyle diseases that are attracting attention in recent years, metabolic syndrome (visceral adiposity syndrome) may be mentioned. The metabolic syndrome refers to a state of exhibiting complex symptoms in which obesity is combined with hypertension, hyperglycemia, hyperlipidemia or the like. A material capable of effectively exerting an effect against such complex symptoms, and particularly, a material capable of effectively controlling the expression of adipokines such as leptin or PAI-1, can be useful in ameliorating the metabolic syndrome.
Patent Document 1: JP-A No. 2001 -172296
Patent Document 2: JP-A No. 2001-120237
Patent Document 3: JP-A No. 2001-299272
Patent Document 4: JP-A No. 2002-53484
Patent Document 5: JP-A No. 2004-161644
Patent Document 6: JP-A No. 2006-1872
Non-Patent Document 1: Ebihara, Ken, et al. , Adiposcience, Vol. 1, No. 3, 259-266 (2004)
Non-Patent Document 2: Yamamoto, Koji, Adiposcience, Vol. 1, No. 3, 273-279 (2004)

### Disclosure of the Invention

### Problems to be Solved by the Invention

An object of the present invention is to provide a new body fat reduction-promoting agent which can solve obesity by promoting the reduction of accumulated fat; a leptin production-promoting agent which can promote the production of leptin in adipocytes; an anorexigenic agent which can solve obesity by suppressing food intake under the leptin production promoting effects; a PAI-1 production-suppressing agent which can suppress the production of PAI-1 in adipocytes; and an anti-thrombotic agent which suppresses thrombus formation under the PAI-1 production suppressive effects.

### Means for Solving the Problems

The inventors of the present invention conducted a search of components exhibiting such effects, among a variety of food candidates, and confirmed a body fat reduction promoting activity, a leptin production promoting activity and a PAI-1 production suppressing activity of a chestnut astringent skin extract, thus completing the invention as described below.

(1) A body fat reduction-promoting agent comprising a chestnut astringent skin extract as an active ingredient.

(2) The body fat reduction-promoting agent according to claim 1, for reducing the body fat accumulated in the viscera.

(3) A leptin production-promoting agent comprising a chestnut astringent skin extract as an active ingredient.

(4) An anorexigenic agent comprising a chestnut astringent skin extract as an active ingredient.

(5) A PAI-1 production-suppressing agent comprising a chestnut astringent skin extract as an active ingredient.

(6) An anti-thrombotic agent comprising a chestnut astringent skin extract as an active ingredient.

### [Effects of the Invention]

The present invention is a very safe body fat reduction-promoting agent, leptin production-promoting agent, anorexigenic agent, PAI-1 production-suppressing agent or anti-thrombotic agent, which is extracted from chestnut astringent skin, a naturally occurring component. In particular, the body fat reduction-promoting agent has an effect of reducing fat that has been accumulated, and thus can directly solve obesity. The body fat reduction-promoting agent is particularly effective in reducing visceral fat.

### Best Mode for Carrying Out the Invention

The present invention is a body fat reduction-promoting agent, leptin production-promoting agent, anorexigenic agent, PAI-1 production-suppressing agent or anti-thrombotic agent, containing as an active ingredient, a component extracted from the astringent skin of chestnut, which belongs to the genus Castanea of the family Fagaceae, for example, Japanese chestnut (Castanea crenata Sieb. Et Zucc), Chinese chestnut, European chestnut, American chestnut and the like, using an appropriate solvent or other extraction medium other than that. Among others, it is preferable to use an extract obtained from the astringent skin derived from Japanese chestnut.

It is known that an extract of chestnut astringent skin has an α-glucosidase inhibitory activity as a physiological action (Patent Document 6 shown above). The document proposes to use the chestnut astringent skin extract as an anti-obesity agent. However, the anti-obesity action based on α-glucosidase inhibition involves suppressing the absorption of sugars from the digestive tract, and thereby suppressing the conversion of taken-in calories to fat. Thus, such action is different in nature from the function of directly reducing the fat stored by adipocytes. Furthermore, as far as the inventors of the present invention conduct an investigation, the chestnut astringent skin extract was not recognized to have a lipase activity inhibitory action.

On the other hand, it has been newly confirmed by the research of the present inventors that the astringent skin extract according to the present invention has an action of reducing the fat accumulated in adipocytes, particularly the fat accumulated in visceral cells. Such action is clearly different from the α-glucosidase inhibitory action, and is to effect direct reduction of the amount of accumulated fat. In addition, it has been newly confirmed by the research of the present inventors that the astringent skin extract according to the present invention has an action of promoting the production of leptin, which is a good cytokine, and an action of suppressing the production of PAI-1, which is a bad cytokine, from adipocytes. Promotion of leptin production brings forth an anorexigenic effect, while suppression of PAI-1 production brings forth an anti-thrombotic effect.

In the present invention, the astringent skin collected from raw chestnuts which have been rid of the brown shells, may be used directly, or this astringent skin in a dried and pulverized state may also be used. Furthermore, the astringent skin recovered from calcined, heated, frozen or dried chestnuts, or from boiled chestnuts, is also acceptable. During the extraction using a solvent that will be described later or the like, it is preferable to use finely pulverized astringent skin.

Preparation of an extract from the astringent skin can be carried out using various extraction methods such as solvent extraction, enzymatic degradation, press extraction, centrifugation and supercritical extraction. In particular, it is preferable to use solvent extraction from the viewpoint of operational convenience.

The solvent used in solvent extraction may be a solvent that is acceptable under the Food Hygiene Law, and for example, water, hydrophilic organic solvents, acetone, hexane and the like can be used. As for the hydrophilic organic solvent, lower alcohols such as methanol, ethanol, propanol, isopropanol and butanol; polyhydric alcohols such as propylene glycol, 1,3-butanediol and glycerin; mixtures thereof, or a mixture of these with water may be mentioned. Without particular limitation, it is preferable to use ethanol or a mixed solution of water and ethanol, from the viewpoint of safety. The concentration of ethanol can be appropriately selected in the range of 5% by volume to 100% by volume, and preferably 50% by volume to 80% by volume.

The extraction treatment may be performed by adding an extraction solvent in an amount of about 0.5 to 4 times by weight based on a weight of chestnut astringent skin, at ordinary pressure or under pressure, at ordinary temperature or in a heated state, while stirring as necessary, for several minutes to several days, preferably for about 10 minutes to several hours, and more preferably about 10 hours to 30 hours. The extraction may be of batch type or reflux type. It is not necessary to carry out the temperature management during the extraction particularly stringently, and the temperature may be approximately in the range of 10 to 80°C.

In order to obtain the extract according to the present invention conveniently and efficiently, an extract liquid according to the present invention recovered after extracting with hydrous ethanol or acetone and then removing the insoluble by filtration or centrifugation, may be directly used, or a soluble fraction obtained by further fractionating the extract liquid with methanol or the like, may be used. The extract according to the present invention may also be obtained by further removing the solvent from the extract liquid by a known means such as distillation under reduced pressure, spray drying or freeze-drying.

A product obtained by further purifying the extract liquid or the extract by applying appropriate purification treatments such as, for example, column chromatography using an adsorbent such as silica gel, ion-exchange resin or activated carbon, or solvent fractionation, may also be used.

The body fat reduction-promoting agent, leptin production-promoting agent, anorexigenic agent, PAI-1 production-suppressing agent or anti-thrombotic agent according to the present invention can be prepared into a composition for reducing body fat, for promoting leptin production, for suppressing food intake, for suppressing PAI-1 production or for anti-thrombosis, by incorporating various raw materials or components, as long as such incorporation is not contradictory to the purpose of the present invention. For example, excipients, desiccants, antiseptics, reinforcing agents, thickening agents, emulsifiers, antioxidants, sweetening agents, acidulants, seasoning agents, colorants, flavors and the like, which are used in food products or pharmaceutical products, can be all incorporated together with the body fat reduction-promoting agent, leptin production-promoting agent, anorexigenic agent, PAI-1 production-suppressing agent or anti-thrombotic agent of the present invention.

Furthermore, those known materials which are used for the prevention or treatment of obesity, diet or slimming, may also be used in combination with the body fat reduction-promoting agent, leptin production-promoting agent, anorexigenic agent, PAI-1 production-suppressing agent or anti-thrombotic agent of the present invention. For example, plant extracts such as a fruit peel extract of Garcinia cambogia, a grape seed extract, polyphenol in fruits such as apple, a hawthorn fruit extract, a guava leaf extract, a Gymnema sylvestre leaf extract and a Ginkgo leaf extract; enzyme inhibitors such as a lipase inhibitor, and α- and β-amylase inhibitors; L-carnitine, a green algae extract, a brown algae extract, and the like may be mentioned as the examples of combination components.

As an embodiment of the composition added with the above-mentioned excipients or known materials, an edible composition or a pharmaceutical composition is preferred.

The edible composition may be prepared by adding the body fat reduction-promoting agent, leptin production-promoting agent, anorexigenic agent, PAI-1 production-suppressing agent or anti-thrombotic agent of the present invention, to a beverage such as a fruit drink, a soft drink or tea; a food product such as soup or jelly; a dairy product; and others. Also, if necessary, excipients such as dextrin, lactose, starch and powdered cellulose; nutrients such as vitamins, minerals, oils and fats, proteins and saccharides; or additives such as colorants and flavors, are incorporated, and the mixture is processed to powders, granules, pellets, tablets or the like, or coated with gelatin or the like, followed by molding into capsules. Then, the preparation may be used as nutrition supplement food or as health food.

The amount of incorporation of the body fat reduction-promoting agent, leptin production-promoting agent, anorexigenic agent, PAI-1 production-suppressing agent or anti-thrombotic agent of the present invention in the edible composition may vary depending on the form of the agent or the form of the composition (extract liquid, dry powder, or the like), but preferably, the amount may be appropriately adjusted in the range of 0.01 to 20% by weight per weight percent of the edible composition.

In order to prepare a pharmaceutical composition, excipients or additives appropriately according to the formulation may be added to the body fat reduction-promoting agent, leptin production-promoting agent, anorexigenic agent, PAI-1 production-suppressing agent or anti-thrombotic agent of the present invention, as needed, and the mixture may be processed by a conventional method to obtain preparations such as tablets, capsules, granules, powders or injectable preparations. The amount of incorporation of the body fat reduction-promoting agent, leptin production-promoting agent, anorexigenic agent, PAI-1 production-suppressing agent or anti-thrombotic agent of the present invention in the pharmaceutical composition is dependent on the form of the agent or the form of the composition, or the administration route, but preferably, the amount may be appropriately adjusted in the range of 0.1 to 60% by weight per weight percent of the composition. The amount of application in the case of preparing the body fat reduction-promoting agent, leptin production-promoting agent, anorexigenic agent, PAI-1 production-suppressing agent or anti-thrombotic agent of the present invention into an edible composition or a pharmaceutical composition, can be appropriately selected in accordance with the weight and age of the living body to be applied, items in the diet, the form of application, or the like.

Hereinafter, the present invention will be described in more detail with reference to Examples, but the present invention is not intended to be limited to these Examples.

### Brief Description of the Drawings

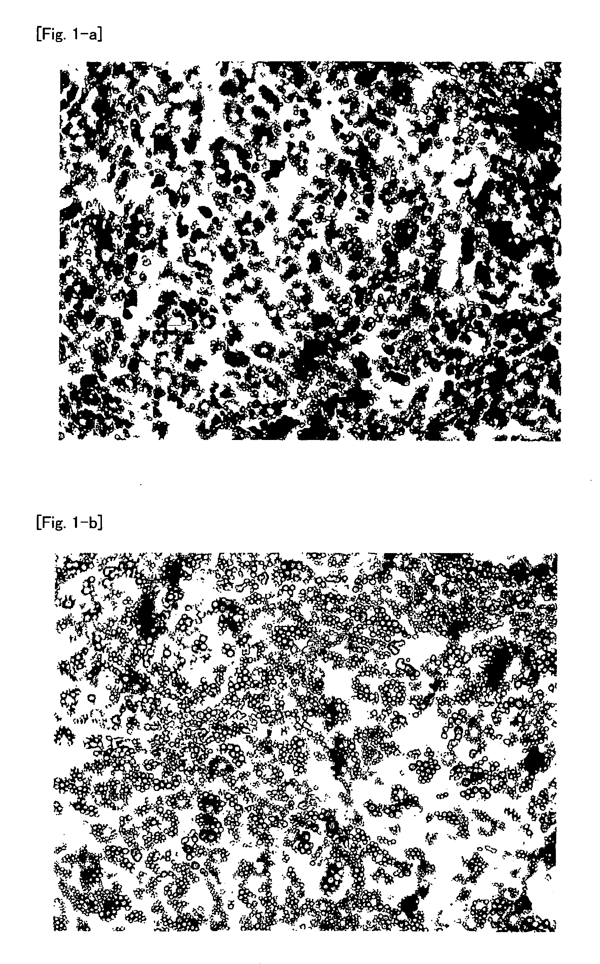

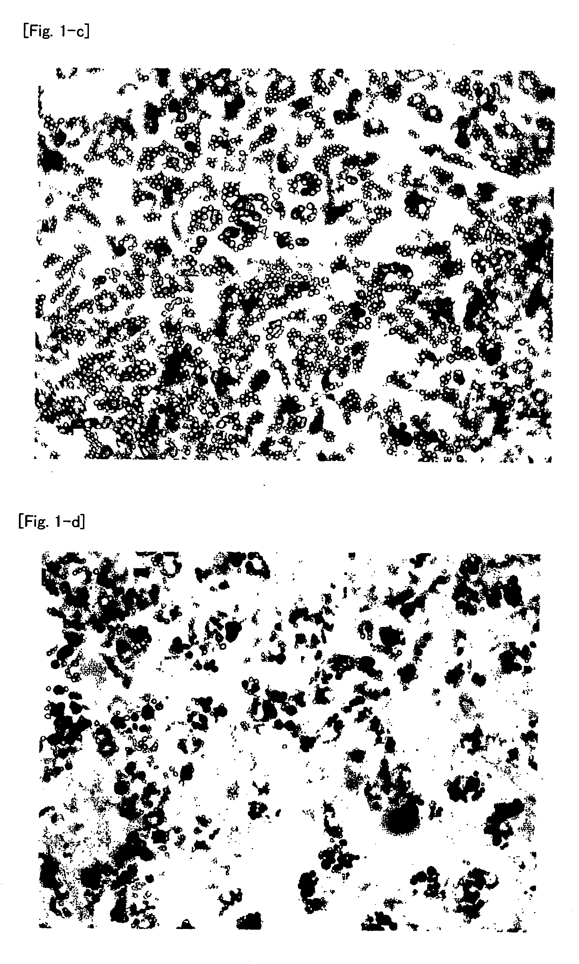

Fig. 1-a is a diagram showing the results of Oil Red staining performed on preadipocytes cultured in wells added only with the visceral adipocyte differentiation medium.
Fig. 1-b is a diagram showing the results of Oil Red staining performed on preadipocytes cultured in wells added with 0.001 mg of a chestnut astringent skin extract.
Fig. 1-c is a diagram showing the results of Oil Red staining performed on preadipocytes cultured in wells added with 0.01 mg of a chestnut astringent skin extract.
Fig. 1-d is a diagram showing the results of Oil Red staining performed on preadipocytes cultured in wells added with 0. 1 mg of a chestnut astringent skin extract.
Fig. 2 shows the results obtained by eluting the dye after Oil Red staining and measuring the absorbance.
Fig. 3 shows changes in the body weight when the body fat reduction-promoting agent was taken.
Fig. 4 is a graph showing the amount of production of leptin on the 6th day after the initiation of culture.
Fig. 5 is a graph showing the amount of production of PAI-1 on the 6th day after the initiation of culture.

### EXAMPLES

### <Example 1>

Astringent skin was peeled off from the raw chestnuts obtained by removing brown shells from Japanese chestnuts belonging to the genus Castanea of the family Fagaceae. To 100 g of the astringent skin, 200 ml of a liquid mixture of water/ethanol (70 vol% ethanol solution) was poured, and extraction was performed at 50°C or below for 15 hours. Subsequently, filtration was performed to separate the filtrate and the residue. The filtrate was concentrated in an evaporator and then freeze-dried, to obtain 4 g of a chestnut astringent skin extract powder.

### <Test Example 1>

A test was performed on the fat content in adipocytes using the "VAC Visceral Adipocyte Culture Kit" of Cell Garage Co., Ltd. according to the protocol of the kit, and using rat visceral preadipocytes and the visceral adipocyte differentiation medium. Rat visceral preadipocytes were thawed at 37°C, the visceral preadipocytes were transferred to a 15-mL centrifuge tube, and 10 mL of the visceral adipocyte differentiation medium was added thereto and the mixture was suspended. After the suspension, centrifugation (4°C, 1000 rpm, 5 minutes) was performed, and the supernatant resulting from the centrifugation was removed. Then, 10 mL of the visceral adipocyte differentiation medium was added, and the mixture was suspended and subjected to centrifugation (4°C, 1000 rpm, 5 minutes). The supernatant resulting from the second centrifugation was removed. After the removal of the supernatant, 12.5 mL of the visceral adipocyte differentiation medium was added to the visceral preadipocytes, and thus a cell suspension (2.4 × 10⁵ cells/mL) was prepared.

500 µL of the cell suspension was placed (inoculated) into each well of a 24-well plate, and culture (37 ± 0.5°C, CO₂ concentration 5%) was initiated. On the first day after the initiation of culture, 500 µL of the visceral adipocyte differentiation medium was added to each well, and the culture was further continued. On the second day after the initiation of culture, the medium was exchanged, and 1 mL of new visceral adipocyte differentiation medium was added. Also, the body fat reduction-promoting agent (powder) prepared in the Example was added to a concentration of 0.001 mg/well, 0.01 mg/well or 0.1 mg/well. The culture was continued for 7 days. As a control, wells not added with the body fat reduction-promoting agent (powder) were also provided.

A test for measuring the fat content in cells was performed using the "Lipid Assay Kit" of Cell Garage Co., Ltd. according to the protocol of the same kit, and using Oil Red O stock solution, a fixing solution and an extract liquid.

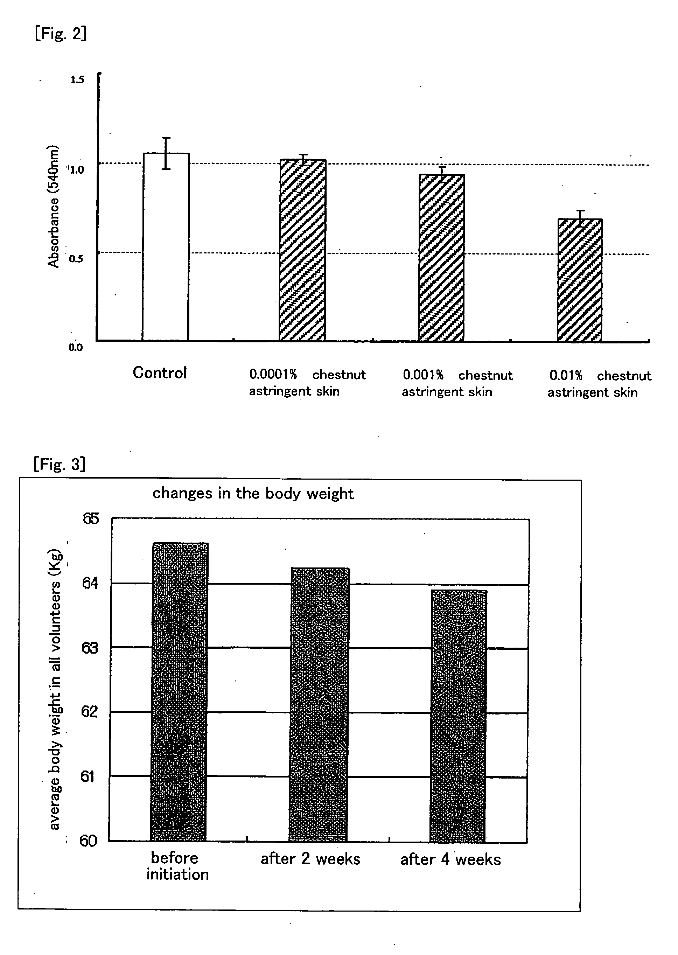

On the last day of culture, the medium was removed from the wells, and 500 µL of a fixing solution prepared by filtering a mixture solution of Oil Red O stock solution: distilled water = 6 : 4, which had been left to stand for 10 to 15 minutes at room temperature, through a 0.5-µL syringe filter, was added to each well. The plate was left to stand for 15 minutes at room temperature, and Oil Red staining was performed. After the staining, a washing operation was performed several times using distilled water (Fig. 1). Subsequently, 500 µL of the extract liquid was added to each well, the dye was eluted, and then the absorbance (540 nm) of the eluate was measured with a plate reader (Fig. 2).

As a result, there was observed a volume-dependent reduction of the fat content in the cells in those wells added with the chestnut astringent skin extract of the present invention.

### <Test Example 2>

Twenty male and female volunteers (age of 23 to 59) were prescribed to take one tablet containing 0.15 g of the body fat reduction-promoting agent (powder) of the present invention prepared in Example 1 for every 80 g of hydrocarbon content in food taken in, before every meal for 4 weeks. Changes in their body weights over the time period were monitored. The results (changes of the average body weight in all volunteers) are presented in Fig. 3. The hydrocarbon content in food was determined on the basis of the estimation made with reference to the "Food Table for Hydrocarbons in Foods" (Richard Heller, et al., "Low Carb Diet, " 4th Edition, published byNeko Publishing Co., Ltd. (2002)).

As is clear from Fig. 3, when the body fat reduction-promoting agent of the present invention was continuously taken, reduction of body weight was observed.

Furthermore, among the volunteers, three males having a body fat percentage of 25% or more and three females having a body fat percentage of 30% or more were selected, and their body fat percentage was measured at the initiation and completion of the test, respectively. As a result, the body fat percentage decreased by 1.06% in average (body weight decreased by 0.2 kg) in the three males, while the body fat percentage decreased by 0.33% in average (body weight decreased by 1. 33 kg) in the three females.

### <Test Example 3>

Three-dimensional culture was performed for about two weeks using the "VAC Visceral Adipocyte Culture Kit" of Cell Garage Co., Ltd., by collagen gel-embedding the rat visceral preadipocytes enclosed in the same kit, and adding the visceral adipocyte differentiation medium enclosed in the kit.
Specifically, first, the rat visceral preadipocytes were thawed at 37°C, the visceral preadipocytes were then transferred to a 15-mL centrifuge tube, and 10 mL of the visceral adipocyte differentiation medium was added thereto and the mixture was suspended. After the suspension, centrifugation (4°C, 1000 rpm, 5 minutes) was performed, and the supernatant resulting from the centrifugation was removed. Then, 10 mL of the visceral adipocyte differentiation medium was added, and the mixture was suspended and subjected to centrifugation (4°C, 1000 rpm, 5 minutes). The supernatant resulting from the second centrifugation was removed. After the removal of the supernatant, 6.4 mL of the collagen solution (cell matrix Type I-A) enclosed in a collagen gel culture kit (manufactured by Nitta Gelatin, Inc.), 0.8 mL of a concentrated medium (Ham's F-12), and 0.8 mL of a buffer solution for reconstitution were added to the visceral preadipocytes, and thus a cell suspension (3.75 × 10⁵ cells/mL) was prepared.

300 µL of the cell suspension was placed (inoculated) into each well of a 12-well plate, 1.4 mL of the visceral adipocyte differentiation medium was added, and culture (37 ± 0.5°C, CO₂ concentration 5%) was initiated. On the second day after the initiation of culture, the medium was exchanged, and 1. 4 mL of new visceral adipocyte differentiation medium was added. In addition, the chestnut astringent skin extract powder prepared according to the Example was added to each well to achieve a concentration of 0.001% or 0.01%, and the culture was continued (n=3). As a control, wells not added with the chestnut astringent skin extract powder were also provided.

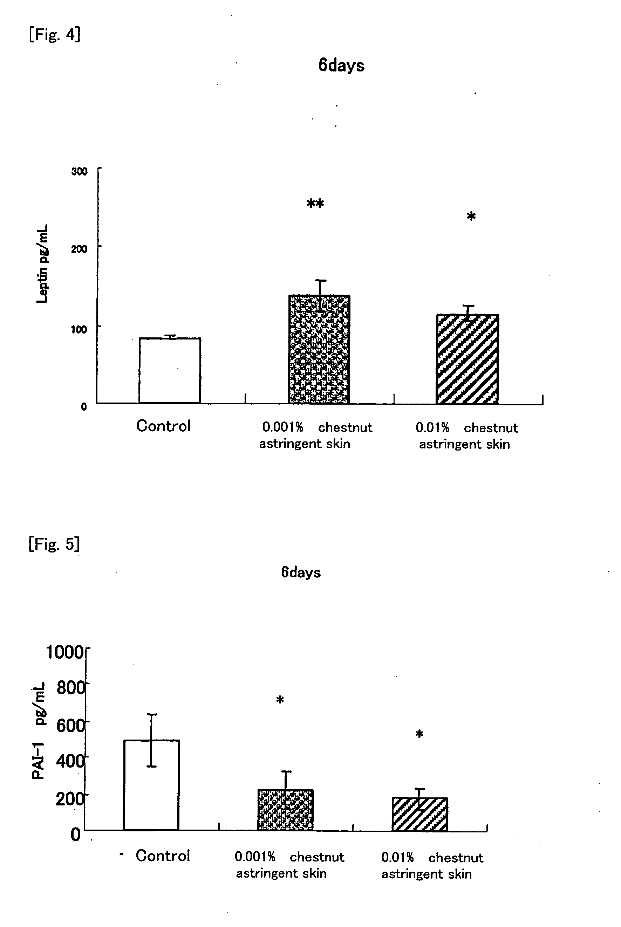

After 6 days from the initiation of culture, the amount of adipokines in the culture supernatant recovered at the time of exchanging the medium was measured using a commercially available kit (RAT ADIPOCYTE LINCOplex KIT). Specifically, 25 µL of the culture supernatant was incubated at 4°C for 16 hours, together with a panel of microspherical beads, each coated with antibodies specific to leptin and PAI-1, which were species of adipokine. After the incubation, the beads mixture was washed, and was incubated for 1 hour at room temperature, together with the detection antibody cocktail enclosed in the same kit. Subsequently, streptavidin-phycoerythrin was further added, and the mixture was incubated for 30 minutes at room temperature. At the same time, the incubation of a standard prepared by serial dilution was performed in the same order. Subsequently, the bead suspension was washed, and resuspended with a buffer agent to quantify the adipokine concentration, for the reading with a Luminex System (Luminex (registered trademark) 100). A comparison with the control was made using the Dunnett's test, and the case where the risk rate (p) was 5% or less, was determined to have a significant difference (*: p < 0.05, **: p < 0.01 vs Control (Dunnett's multiple comparison test)). As a result, it was confirmed that the chestnut astringent skin extract significantly promoted leptin production (Fig. 4), and significantly suppress PAI-1 production (Fig. 5).

As discussed in the above, it was confirmed that when a chestnut astringent skin extract was used, accumulation of visceral fat, which poses a problem particularly in the metabolic syndrome and the like, was resolved, and the body weight could be reduced; that leptin production in adipocytes could be promoted; and that production of PAI-1 in adipocytes could be suppressed. Therefore, the chestnut astringent skin extract can be beneficially used as a body fat reduction-promoting agent, a leptin production-promoting agent, an anorexigenic agent; a PAI-1 production-suppressing agent, and an anti-thrombotic agent, and is effective in the treatment or amelioration of various symptoms observed in the metabolic syndrome or obesity.

## Claims

1. A body fat reduction-promoting agent comprising a chestnut astringent skin extract as an active ingredient.

2. The body fat reduction-promoting agent according to claim 1, for reducing the body fat accumulated in the viscera.

3. A leptin production-promoting agent comprising a chestnut astringent skin extract as an active ingredient.

4. An anorexigenic agent comprising a chestnut astringent skin extract as an active ingredient.

5. A plasminogen activator inhibitor-1 production-suppressing agent comprising a chestnut astringent skin extract as an active ingredient.

6. An anti-thrombotic agent comprising a chestnut astringent skin extract as an active ingredient.
